# EUROPEAN PATENT APPLICATION

(11) **EP 3 293 595 A1**
(43) Date of publication of application: **14.03.2018**
(21) Application number: 17190834.6
(22) Date of filing: 13.09.2017
(51) Int. Cl.: G05B 23/02

(54) **AN INTEGRATED SYSTEM AND METHOD FOR ONLINE MONITORING AND OFFLINE TESTING IN A TEXTILE UNIT**

(30) Priority: 13.09.2016 IN 201641031200
(71) Applicant: Premier Evolvics PVT. Ltd., 641 062 Coimbatore (IN)
(72) Inventor: VARADARAJAN, Srinivasan, 641005 Coimbatore (IN); GURUPRASATH, Krishnamoorthy, 641015 Coimbatore (IN)
(74) Representative: Frei Patent Attorneys

(57) **Abstract**

A system for yarn production monitoring, comprising an online quality monitoring device, and an offline testing system (100),
- wherein the online quality monitoring device comprises a sensor (3),
o wherein the sensor (3) is adapted to be mounted at a production position of a textile manufacturing unit,
o wherein the sensor (3) is adapted to continuously measure a parameter of a production process of a production position at which it is mounted,
o wherein the online quality monitoring device is adapted to generate quality data of a production process using results of measurements by the sensor (3); and

- wherein the system is adapted to transmit data comprising quality data from the online quality monitoring device to the offline testing system (100).

Furthermore, a combination comprising such a system; and a method relating to such a system.

## Description

The present invention relates to systems, combinations and methods for yarn production monitoring. Yarn is typically produced using textile manufacturing units, e.g. ring spinning machines. These machines can comprise a number of production position. Each production position can be designed to process a single thread of yarn. A factory can comprise multiple textile manufacturing units and each such unit can comprise multiple production positions. Therefore, a system and method for yarn production monitoring that allow for an efficient surveillance of the manufacturing process is desirable.

US 5,381,340 discloses a quality control system in a spinning mill for producing packages via at least one or more of the steps of mixing and blowing, combing, drawing, roving, fine spinning and winding. The Mill contain several different processing machines for performing several different processing steps to produce several different intermediate products. Each processing machine is provided with sub-processor for monitoring production information associated with the processing machine and quality information associated with the intermediate product produced by the processing machine. The quality and production information from the various sub-processors are directed to main processor, which records a history of the processing of various intermediate products.

US 5,517,404 discloses a spinning mill with a master process control computer for at least one group of machines, whereby each machine of the group is provided with its own machine control unit which controls the actuators of the machine. At least one network is provided for bidirectional communication between the computer and each machine of the group. During the operation of the plant, master control instructions from the process control computer are supplied via network to the machine control units. Each machine control unit forward the control instructions to the actuators controlled by the machine control unit, whereby control instructions, if required, are converted by the machine control unit into signals which are suitable for the actuators.

US 2006/007/9216A discloses a system comprising control computers for controlling and detecting the operating data e.g. the speed, stoppage, cause of stoppage etc. for each textile machine, and a central computer for determining the production data. The control computer and the central computer respectively comprise means for wireless signal transmission, and form with a respectively associated transmission unit, a radio network for signal transmission according to the WLAN or Blue tooth standard.

WO 1993009279 A1 discloses a spinning facility with a process-control computer for at least one group of machines, each machine in the group being fitted with its own control unit which controls all operation of the machine. At least one communication network is provided for duplex communication between the computer and each machine in the group. In this system, the sensors have been uprated and programmes adapted in order to be able to indicate correlations between operating performance and factors which influence it.

The present invention provides an improved system, combination and method for yarn production monitoring according to the independent claims. A possible improvement thereof is to provide a system and method for yarn production monitoring that allows for identifying and solving production problems with increased efficiency.

In some embodiments, a system for yarn production monitoring comprises a sensor. The sensor can be adapted to measure a parameter. The parameter can be a parameter of a yarn, more specifically of a thread of yarn, processed in a textile manufacturing unit. The sensor can be adapted to continuously measure a parameter of a production process of a production position. The sensor can be a multi-sensor and comprise multiple sensor heads. The sensor can be adapted to measure one or more, in particular two or more, parameters.

The sensor can be arranged at, in particular mounted on, a production position of a textile manufacturing unit, e.g. between a delivery roller and a lappet hook of that production position. The sensor can be adapted to continuously measure a parameter of a production process of a production position at which it is mounted. A plurality (e.g. two or more) of sensors can be arranged at a plurality (e.g. two or more) of production positions of one or more textile manufacturing unit.

In some embodiments, a system for yarn production monitoring comprises an online quality monitoring device. The online quality monitoring device can be adapted to monitor a textile manufacturing unit online, i.e. it can be adapted to monitor the unit while it is producing (e.g. the online quality monitoring device monitors the yarn while it is being produced in the unit).
The online quality monitoring device can comprise a sensor. In particular, the online quality monitoring device can comprise a plurality of, e.g. two or more, sensors. The online quality monitoring device can comprise further units, e.g. a data processing unit and/or a data collection unit. The data processing unit and/or data collection unit can e.g. be a computer.

The online quality monitoring device can be adapted to generate data. The online quality monitoring device can be adapted to generate quality data, i.e. data relating to a quality of yarn being produced in a production position of a textile manufacturing unit. The online quality monitoring device can be adapted to generate quality data using results of a measurement of a sensor. The online quality monitoring device can be adapted to generate quality data using measurements of two or more sensors. The online quality monitoring device can be adapted to continuously generate quality data.

In some embodiments the online quality monitoring device comprises a sensor and the sensor generates quality data. In particular, the online quality monitoring device can be adapted to generate quality data of a production process using the results of a measurement of that sensor.

In some embodiments, a system for yarn production monitoring comprises an online quality monitoring device that comprises a plurality of sensors. In some examples of a system for yarn production monitoring in combination with one or more textile manufacturing units, the plurality of sensors are mounted on a plurality of production positions. For example, each production position of a textile manufacturing unit can comprise the same number of sensors; e.g. one, two, three or more.

In some embodiments the online quality monitoring device comprises a sensor and a processor. The processor can be adapted to process data, in particular quality data provided by the sensor. The processor can be arranged within the sensor or at the sensor. The processor can be adapted to process data provided by two or more sensors. The processor can be comprised in a data collection unit.

In some embodiments, the online quality monitoring device can be adapted to receive data from a sensor. E.g. the online quality monitoring device can comprise receiving means for receiving data from a sensor (such as a cable or a unit for wireless transmission). The receiving means can e.g. be comprised in a data collection unit.

In some embodiments, the online quality monitoring device can be adapted to transmit data (in particular quality data received from a sensor). E.g. the online quality monitoring device can comprise transmission means for transmitting data (e.g. a cable or a unit for wireless reception).

In some embodiments, the online quality monitoring device can be adapted to transmit data in that the sensors of the online quality monitoring are adapted to transmit data. E.g. one or all sensors can comprise transmission means for transmitting data (e.g. interface for a cable connection or a unit for wireless reception).

In some embodiments, the online quality monitoring device is adapted to transmit data (e.g. data comprising quality data), e.g. to an offline testing system.

In some embodiments, the online quality monitoring device is adapted to transmit data if a parameter is outside a pre-defined set of values (e.g. tolerance limits). This parameter can be a parameter measured by a sensor or a derived parameter (e.g. a parameter that is calculated using measured parameters; the calculation can for example use a function depending on measured quality parameters, other measured parameters and/or pre-defined values, such as calibration data). The pre-defined set of values can be an interval. The pre-defined set of values can be the union of multiple compact sets (e.g. closed intervals). The parameter can be a multi-value parameter (vector etc.) and the pre-defined set of values can be a subset of possible multi-value parameters (e.g. a union of compact subsets of a vector space). The pre-defined set of values can be a set which is defined by a user. The pre-defined set of values can be a set which was automatically defined by the system. The pre-defined set of values can be the results of a statistic computation. The pre-defined set of values can be calculated according to a distribution (e.g. the standard deviation and/or confidence intervals of the distribution). The pre-defined set of values can be a set which is defined by absolute values. The pre-defined set of values can be a set which is defined by relative values. The pre-defined set of values can be calculated using results of current and/or past measurements.

In some embodiments, the system for yarn production monitoring can comprise an offline testing system The offline testing system can be adapted for testing (e.g. analyzing) samples of yarn and/or coordinating such testing. The offline testing system can comprise one or more offline testing instruments for testing (e.g. analyzing and/or investigating) samples of yarn. The offline testing system can comprise a server, e.g. for coordinating a testing of samples of yarn. The server can be adapted for collecting and/or analyzing data, e.g. data that is provided by an online quality monitoring device as well as data provided by offline testing instruments.

In some embodiments, the system for yarn production monitoring can comprise an offline testing instrument. In particular, the system can comprise two or more offline testing instruments. One or more offline testing instruments can be comprised in an offline testing system. In particular, the offline testing system can be one or more offline testing instruments. An offline testing instrument can be adapted to test (e.g. to analyze and/or investigate) a sample of textile material (e.g. yarn, silver, roving etc.) offline, i.e. it can be adapted to test the sample while not while it is produced (e.g. when the sample is taken out of the yarn resp. out of the production position). An offline testing instrument can e.g. comprise an evenness tester, a tensile tester, a yarn classification instrument and/or a twist tester.

An evenness tester is an instrument for measuring the evenness properties of a textile material. Evenness testers can be adapted to measure additional properties, e.g. such as hairiness, hair count, linear density, foreign fibres etc. An evenness tester can e.g. comprise capacitance sensors for measuring evenness properties and optical sensors of various configurations to measure the hairiness, hair count and foreign fibres.

A tensile tester is an instrument for testing the tensile properties of yarn and fabrics. A tensile tester can comprise two clamps, which are designed to grip the test material (e.g. a sample of yarn). A first clamp can be designed to move in a direction away from a second clamp. This movement can create tension by stretch for the textile materials, and at a certain stress, the test material breaks. The force required to break the textile material and the elongation can be used to derive various tensile properties.

A yarn classification system is an instrument for testing and/or classifying yarn faults, foreign fibres etc. A yarn classification system can comprise capacitance sensors, which e.g. can be designed to measure the mass faults. A yarn classification system can comprise optical sensors, which e.g. can be designed to measure the foreign fibre faults.

A twist tester is an instrument for measuring the amount of twist imparted in yarns. A twist tester can be adapted to determine a number of rotations of a handle of the twist tester needed to untwist the yarn fully; this number can be considered as the basis for measuring twist properties.

In some embodiments, the system for yarn production monitoring is adapted to transmit data, in particular data comprising quality data, from an online quality monitoring device to an offline testing system (e.g. an offline testing instrument of an offline testing system). For example, the system for yarn production monitoring can be adapted to transmit data from the online quality monitoring device to the offline testing system if a parameter (e.g. a parameter measured by one or more sensors and/or a parameter derived therefrom) is outside a pre-defined set of values. The data to be transmitted can comprise quality data (e.g. a thickness or a periodic fault of yarn produced in a specific production position) and/or other data (e.g. machine related data, such as an indication of the specific production position at which a suspicious parameter has occurred).

A suspicious parameter, i.e. a parameter that is outside its acceptable set of values, is also referred to as a fault in the production process. If a fault is detected it is preferable to find the cause (also referred to as the "root" or the "root cause") of the fault as soon as possible and find a way of remedying that cause. The transfer of online data to an offline testing system can allow for a more efficient finding of a cause of and/or a possible remedy for a fault.

The parameters measured by the one or more sensors of an online quality monitoring device and/or the data generated by an online quality monitoring device (e.g. by a sensor thereof) can relate to at least one of the following features of yarn:
- evenness of the yarn,
- thick places of the yarn,
- thin places of the yarn,
- neps in the yarn,
- objectable yarn faults,
- hairiness of the yarn,
- yarn count,
- twist of the yarn, and/or
- yarn classification distribution.
All of these examples can be considered as quality data of the production process, i.e. the data relating to a quality of produced yarn. Data that is considered to not be quality data can e.g. relate to the machinery as such (e.g. machine details and/or an indication of a specific production position).

The term "unevenness" refers to the yarn unevenness. It can e.g. be measured by using capacitance sensors. The result can e.g. be expressed in terms of percentage either as Unevenness % or coefficient of unevenness ("CV%"). The coefficient of unevenness (or irregularity of yarn) can e.g. measured relative to a basic cut length of 1cm; or at larger cut length, such as 1m, 3m, 10m, 50m or 100m.

The term "thick places" refers to parts of a yarn that are unusual thick, i.e. thicker than a specified sensitivity level. The sensitivity level can be defined in absolute or relative terms. Possible thresholds can e.g. be defined as deviation from an average level, e.g. +25%, +35%, +50%, +75% and +100% relative to an average yarn thickness. Yarn thickness can e.g. be measured by using capacitance sensors (e.g. the capacitive sensors of an evenness tester). The result can e.g. be expressed in terms of thick places per km.

The term "thin places" refers to parts of a yarn that are unusual thin, i.e. thinner than a specified sensitivity level. The sensitivity level can be defined in absolute or relative terms. Possible thresholds can e.g. be defined as deviation from an average level, e.g. -25%, -30%, -40%, -50% and -60% relative to an average yarn thickness. Yarn thickness can e.g. be measured by using capacitance sensors (e.g. the capacitive sensors of an evenness tester). The result can e.g. be expressed in terms of thin places per km.

The term "neps" refers to entanglements or knots of fibers. Neps can, similarly to thick places, be characterized with respect to a specified sensitivity level. The sensitivity level can be defined in absolute or relative terms. Possible thresholds can e.g. be defined as deviation from an average level, e.g. +100%, +140%, +200%, +280% and +400% relative to an average yarn thickness. Neps can e.g. be measured by using and optical sensor or capacitive sensor of an evenness tester. The result can e.g. be expressed in terms of neps per km.

The term "objectionable faults" refers to the number of objectionable faults in a yarn, i.e. undesired faults which affect the fabric appearance significantly (e.g. a very big thick place/neps) and should not be allowed in the production processes. Objectionable faults can e.g. be determined/measured by using capacitance and/or optical sensors (e.g. capacitive and/or optical sensors of a yarn classification system). The result can e.g. be expressed in terms of objectionable faults per 100 km.

The term "yarn hairiness" refer to the amount of protruding hairs from the surface of the yarn. It can e.g. be measured by using optical sensors (e.g. the optical sensors of an evenness tester). The result can e.g. be expressed in terms of Hairiness Index ("Hi") or in terms of Count of Hairs for a pre-defined unit length (e.g. Hairs per 100 m).

The term "yarn count" refers to the linear density of yarn. It can e.g. be measured by using a cut- and-weight-method and/or by using automatic yarn count measurement module (e.g. a yarn count measurement module of an evenness tester. The result can e.g. be expressed in terms of direct systems like Tex, Denier and/or in terms of indirect systems like Nec, Nm.

The term "yarn twist" refers to the amount of twist imparted in yarn. It can e.g. be measured using twist testers. The result can e.g. be expressed in terms of twist per inch or twist per metre. The term "yarn classification" refers to the classification of objectionable and non-objectionable faults, e.g. in a traditional classification chart for thick and thin places of a yarn. Yarn classification can e.g. be determined / measured by using a yarn classification system, which e.g. can be based on capacitance and/or optical sensors. The result can e.g. be expressed graphically in a yarn classification chart showing the number of fault classes in a pre-defined matrix.

In some embodiments, the system for yarn production monitoring comprises a data collection unit. The data collection unit can be adapted to collect data of a sensor, in particular a plurality of (e.g. two or more) sensors. The data collection unit can be adapted to collect data of all sensors of the system for yarn production monitoring associated with a single textile manufacturing unit (e.g. of all sensors mounted to the production positions of that textile manufacturing unit). The data collection unit can be adapted to collect data from multiple (e.g. two or more) textile manufacturing units. The data collection unit can comprise means for receiving data from one or more sensors. A data collection unit can be comprised in an online quality monitoring device. A data receiving unit can be comprised in an offline testing system. A data collection unit can comprise indication means, in particular visual indication means.

In some embodiments, the system for yarn production monitoring comprises a data receiving unit. The data receiving unit can be adapted to receive/collect information from one or more data collection units. The data receiving unit can be adapted to collection information from multiple (e.g. two or more) data collection units. In particular, the data receiving unit can be adapted to collect information from all data collection units associated with an online quality monitoring device. A data receiving unit can be comprised in an offline testing system. A data receiving unit can be comprised in an online quality monitoring device. A data receiving unit can comprise indication means, in particular visual indication means.

In a combination of multiple (e.g. two or more) textile manufacturing units and a system for yarn production monitoring, each of the textile manufacturing units can be associated with a data collection unit and a data receiving unit can be adapted to collect information from all the data collection units of the system. Data concerning the quality of a textile material being produced can be collected by a sensor, then be transmitted to the data collection unit of the textile manufacturing unit at which the sensor is mounted, then be transmitted to the data receiving unit, and then transmitted to an offline testing system, e.g. to a server thereof and/or an offline testing instrument thereof. Data transmitted to a server of the offline testing system can be transmitted from the server to an offline testing system. The decision that a parameter is faulty (i.e. outside a pre-defined set of acceptable values) and/or that data concerning that faulty parameter should be transmitted, can be taken at various stages, e.g.
- by the sensor (e.g. by an processor of the sensor),
- by the online quality monitoring device (e.g. by an sensor of the online quality monitoring device),
- by the data collection unit,
- by the data receiving unit, and/or
- by the server.

In some embodiments all data generated by the online quality measurement device is sent to a server of the offline testing system. The server or another unit of the offline testing system can be designed to decide that a parameter is faulty (e.g. is outside a pre-defined set of values) and that data concerning this fault should be sent to one or more offline testing instruments of the offline testing system. The server and/or the other unit of the offline testing system can be designed to decide to which offline testing instruments of the offline testing system which kind data should be sent. For example, the server and/or the other unit may detect a specific fault and decide that an analysis by a subset of the available instruments is sufficient to likely determine the source of and/or a remedy for a fault. In this case, the server can be designed to transmit the data not to all available instruments, but only to this specific subset of available instruments.

An offline testing instrument can comprise indication means to indicate to a user that an analysis should be performed at this instrument.

Data can be transferred to computers of offline testing instruments. Data can be transferred to computers of offline testing instruments by a sensor, by an online quality measurement device, by a data collection unit, by a data receiving unit and/or by a server of the offline testing system.

In some embodiments, the system for yarn production monitoring comprises an online quality monitoring device and an offline testing system. The online quality monitoring device comprises a sensor, which is adapted to be mounted at a production position of a textile manufacturing unit and is further adapted to continuously measure a parameter of a production process of a production position at which it is mounted. The online quality monitoring can comprise a plurality (e.g. two or more) of such sensors. The online quality monitoring device is adapted to generate quality data of a production process, e.g. by using results of measurements by the sensor. The system is adapted to transmit data comprising quality data from the online quality monitoring device to the offline testing system.

In some embodiments, the system is adapted to transmit data comprising quality data from the online quality monitoring device to the offline testing system if a parameter is outside a pre-defined set of values. Such a system can benefit a user in that his attention, e.g. immediately, can be drawn to the production position in question and/or samples of yarn produced therein (e.g. by an indication means). This can enable the user to, e.g. immediately, analyse the cause of a defect (i.e. the parameter that is outside a pre-defined set of values) signalled by the online quality monitoring device, which can allow the user to e.g. initiate suitable corrective actions. The system can be adapted to transmit the data via a data collection unit, a data receiving unit and/or a server.

In some embodiments, the system is adapted
- to indicate (e.g. by an indication means) to a user that a parameter is outside a pre-defined set of values (e.g. a fault is occurred) at certain production position; and
- to transfer data comprising quality data to an offline testing instrument.
Based on this, the user can initiate that a sample of textile material produced by this production position is extracted from the textile machine unit and brought to the offline testing instrument.

Indication means at the offline testing instrument may be adapted to indicate that the sample should be taken to this specific instrument. At the instrument, a user can examine the sample, e.g. based on the data provided by the online quality monitoring device; and draw conclusions on the source that caused the fault and/or on possible remedies based upon the result of his examination and/or the data provided by the online quality monitoring device. This can allow for an efficient correction of a defect in the production.

The system can be used in combination with one or more textile manufacturing units: the sensors can be mounted at production positions of the textile manufacturing units to monitor yarn being produced at the productions positions. In some embodiments, if the online quality monitoring device (e.g. a sensor or a centralized unit thereof) notices that a monitored parameter of the yarn (which is produced in the textile manufacturing machine) is outside a pre-defined set of values (i.e. a fault has occurred, e.g. that a big slub or a periodic slub occurrence has been measured), the online quality monitoring device (e.g. a sensor or a centralized unit thereof) transmits data to the offline testing system (e.g. to offline testing instrument and/or a server thereof). In some embodiments, the online quality monitoring device continuously transfers data to the offline testing system, e.g. to a server thereof; which e.g. can be adapted to transfer data to offline testing systems if a fault is discovered.

The offline testing system is adapted to analyse a sample of yarn offline (i.e. not while being produced, e.g. when taken outside of the production position). Having data (comprising quality data and possibly other data) transferred to the offline testing system can have multiple advantages:
- For example, it can allow to determine which offline testing should be performed, e.g. with which offline testing instruments a sample of yarn from the production position at which the fault has occurred should be investigated. This decision can be made by a user. Alternatively, this decision can be made by the offline testing system (e.g. a computer thereof), which e.g. can then automatically send the data (and/or a relevant part thereof) to one or more suitable offline testing instruments.
- For example, it can support a user in investigating why a parameter was outside its pre-defined set of values. E.g. the data can support the user in drawing conclusions from the investigation performed by the user at an offline testing instrument. The investigation resp. the drawing of conclusion can as well be performed automatically, e.g. by the offline testing system and/or an offline testing instrument thereof.
Overall, having the data transferred to the offline testing system can support the search for a production problem's cause (and a possible remedy therefor), thereby saving time, money and/or resources.

In some embodiments, the system comprises a data collection unit that is adapted to collect data from a sensor, in particular from two or more sensors. The data collection unit can be adapted to collect data from all the system's sensors mounted at a single textile manufacturing unit. The system can be adapted to transmit the data from the online quality monitoring device to the offline testing system via the data collection unit. The data collection unit can be comprised in the online quality monitoring device. The data collection unit can be comprised in the offline testing system.

In some embodiments, the system comprises a data receiving unit that is adapted to receive data from one or more data collection units, in particular from two or more data collection units. The data collection unit can be adapted to receive data from all data collection units of the system. The data collection unit can comprise means for transmitting data to the offline testing system. The system can be adapted to transmit the data from the online quality monitoring device to the offline testing system via the data receiving unit. The data receiving unit can be comprised in the online quality monitoring device. The data receiving unit can be comprised in the offline testing system.

In some embodiments, the offline testing system comprises an offline testing instrument. An offline testing instrument can comprise an evenness tester, a tensile tester, a yarn classification instrument and/or a twist tester. In particular, an offline testing instrument can be an evenness tester, a tensile tester, a yarn classification instrument and/or a twist tester. The offline testing system can comprise one, two or more testing instruments. The offline testing system can comprise two or more different testing instruments.

In some embodiments, the offline testing system comprises a server. For example, the system can be adapted to transmit data (comprising quality data) from the online quality monitoring device to the server of the offline testing system. The server can be adapted to collect data from a data receiving unit and/or one or more data collection units. The server can be adapted to transmit data, in particular any collected data, to an offline testing instrument. The server can be adapted to analyse data provided by the online quality monitoring device and data provided by the offline testing system (in particular data provided by an offline testing instrument thereof).

In some embodiments, the system is adapted to transmit data. The system can be adapted to transmit data by using wired technologies, e.g. RS 232, RS 422, TCP/IP, and/or CAN (Controller Area Network). The data can be transmitted by using wireless technologies, e.g. Wi-Fi and/or Bluetooth.

In some embodiments, the data that the system is adapted to transmit comprises a machine number, a spindle number, and/or a linear density.

In some embodiments, the system comprises indication means. The system can be adapted to activate the indication means if a parameter is outside a pre-defined set of values. The indication means can comprise digital information means and/or light indication means. The indication means can e.g. comprise a LED or LCD display. The indication means can be adapted to generate a text, an icon and/or an image. In particular, the indication means can be adapted to generate a text, an icon and/or an image comprising a name, icon and/or image of a specific machine component if a fault, in particular a periodic fault, is caused by that specific machine component. The indication means can be comprised in the online quality monitoring device. The indication means can be comprised in and/or mounted at a sensor. The indication means can be comprised in a data collection unit (e.g. wherein the indication means are adapted to indicate faults occurring at any production position associated with that data collection unit). The indication means can be comprised in a data receiving unit (e.g. wherein the indication means are adapted to indicate faults occurring at any production position associated with a data collection unit that is associated with the data receiving unit). The indication means can be comprised in the offline testing system. The indication means can be comprised in a server. The indication means can be comprised in an offline testing instrument.

The system can for example be adapted to notice a fault, in particular a periodic fault, based on the quality data generated by the online quality monitoring device. The system can be adapted to indicate such a fault e.g. to a user (e.g. by an indication means). Based on such indication, a sample of textile material can be extracted from the production position at which the fault has occurred; and the sample can be transported to offline testing instrument for further testing.

The system can be designed to transfer data from the offline testing system to the online quality monitoring device. In particular, the system can be designed to transfer data from an offline testing instrument and/or a server of the offline testing system to the online quality monitoring device. The data transferred to the online quality monitoring system can comprise data generated by the offline testing system and/or by a user using the offline testing system ("offline data"). The data transferred to the online quality monitoring system can comprise information on the causes and/or possible remedies for a fault. The information on causes and/or possible remedies can be based on offline data. The information on causes and/or possible remedies can be based on offline data and on data generated by the online quality monitoring system ("online data").

In some embodiments, the system can be designed to transfer information from the offline testing system to an indication means, in particular an indication means of the online quality monitoring device. For example, the system can be designed to transfer information concerning the causes and/or possible remedies for a specific fault to an indication means. The indication means can be designed to indicate, in particular to display, information concerning causes and/or possible remedies of a specific fault to a user. For example, the indication means can comprise a display that is adapted to display a text, an icon and/or an image that indicates the causes and/or possible remedies for a certain fault (e.g. by displaying a text, an icon and/or an image comprising a name, an icon and/or an image of machine components which cause the fault).

In some embodiments, the system is adapted to transfer offline data to the quality monitoring device, and the evaluation of the causes and/or possible remedies for a specific fault is conducted by the online quality monitoring device and/or a user thereof. This evaluation can be based on offline data (transferred from the offline testing system to the online quality monitoring device) and on online data generated by and available in the online quality monitoring device.

In some embodiments, the system can comprise indication means which are light indication means, such as light bulbs, LEDs, LCD-displays etc. The light indication means can be adapted to illuminate in two or more colors. The light indication means can be adapted to flash in a specific sequence (i.e. pattern). The light indication means can be adapted to flash two or more sequences. One possible sequence in which the light indication means can be adapted to flash is a constant glow.

In some embodiments, the online quality monitoring device comprises means for light indication. The light indication means can be used to alert a user that in (or due to) a certain component of the textile manufacturing unit a (e.g. periodic) fault has occurred. The light indication means can be adapted to show a text, an icon or an image of the component causing the fault. This can allow technicians to quickly attend the defective component. For different defects detected by the system, different color lamp indications can be denoted, e.g. using stable glowing, flashing and/or flashing at various frequencies. The information can also be transferred to and represented visually in the relevant offline laboratory instruments.

The invention further concerns a combination comprising a system for yarn production monitoring, e.g. as described before, and a textile manufacturing unit, wherein the textile manufacturing unit comprises a plurality (e.g. two or more) of production positions (e.g. at which yarn can be produced), the online quality monitoring device comprises a plurality (e.g. two or more) of sensors and the plurality of sensors is mounted on the plurality of production positions (e.g. a specific number of, e.g. one or two, sensors at each of the production positions). The sensors can be adapted to continuously generate quality data of the production process, e.g. in that the sensors are adapted to continuously measure a parameter (in particular two or more parameters) of a production process of a production position at which they are mounted and in that the online quality monitoring device (e.g. the sensors thereof) is adapted to generate quality data of a production process using results of a measurement of the sensor.

In some embodiments, the production positions of the plurality of productions positions comprise rollers for delivering yarn being produced and sensors of the plurality of sensors are mounted at each of the production positions. The sensors can be adapted to continuously measure the quality of yarn being delivered from the rollers.

In some embodiments, the production positions each comprise a lappet hook, which can e.g. be designed for guiding the produced textile material. A sensor can be placed in between the delivery rollers and the lappet hook. A sensor can be arranged at the lappet hook. In particular, a sensor can be placed at a lappet hook and in between the delivery rollers and the lappet hook (e.g. shortly above the lappet hook). A first sensor can be arranged shortly above (from the perspective of the thread flow "shortly before") a lappet hook and a second sensor can be arranged shortly below (from the perspective of the thread flow "shortly after") a lappet hook; the second sensor can e.g. be designed to measure a rotation speed of a spindle.

In some embodiments, the combination comprises two or more textile manufacturing units. The sensors of the online quality monitoring device can be mounted at each of the textile manufacturing units. The combination can comprise two or more data collection units. To each textile manufacturing unit a data collection unit can be associated. In other words, all the sensors mounted at a specific textile manufacturing unit can be adapted to transfer data to a specific data collection unit. Two or more textile manufacturing units can be adapted to transmit data to the same data collection unit. In some embodiments, to each textile manufacturing unit a unique data collection unit is associated. In other words, in such an embodiment there are exactly as many data collection units as textile manufacturing units, and all the sensors mounted at a specific textile manufacturing unit are adapted to transfer data to a specific data collection unit, and the sensors of no two textile manufacturing units are adapted to transfer their data to the same data collection unit

In some embodiments, the combination comprises a data receiving unit, which is adapted to collect data from one or more data collection units. In particular, the data receiving unit can be adapted to collect data from two or more data collection units. The data receiving unit can be adapted to collect data from all data collection units of the combination. The data receiving unit can be adapted to transmit collected data (in particular data collected from data collection unit and/or the sensors) to the offline testing system, in particular to one more offline testing instruments of the offline testing system.

In some embodiments, the combination comprises indication means. The indication means can be adapted to indicate to a user at which production position respectively at which textile manufacturing unit a fault has occurred. This can allow the user to initiate the extraction of a sample from the faulty production position. The indication can comprise an information concerning to which offline testing instrument the sample should be taken. The indication can comprise an information on the nature (e.g. size, amount, weight) of the samples taken. The sample can e.g. be a complete bobbin. The indicated information on the nature can e.g. be determined by the system based on the quality data and for example be adapted to the specific offline testing instruments that the system has determined the sample to be taken. The indication means can be adapted to indicate to a user information on the cause of a fault and/or on a possible remedy for a fault. The information on the cause of a fault and/or on a possible remedy for a fault can be determined by the system and/or a user thereof, e.g. by making use of the online data and the offline data produced with the help of the system.

In some embodiments, the online quality monitoring system comprises a sensor that is positioned on individual delivery points of a textile manufacturing unit in form of a ring frame. The sensor can be adapted to monitor the status of the textile material. In ring frames, the textile material, after delivery from a delivery roller, is further passed through a traveler which is rotating on a ring. The textile material is finally wound on a cop which is rotating over a spindle. The sensor can be adapted to monitor the status of the textile material at one or more positions between the delivery roller and the spindle, e.g. at a lappet hook in between the delivery rollers and the spindle.

In some embodiments, the online quality monitoring device comprises a plurality of sensors mounted on each production position of the textile manufacturing unit. The plurality of sensors can be adapted to continuously measure the quality of the textile material delivered from the rollers. The measured data can be transferred to the data collection unit which is available in each machine. A data receiving unit can be adapted to collect data from multiple data collection units of textile manufacturing units and transfers the data to the computers of the offline testing instruments.

The invention further concerns a method for yarn production monitoring, wherein a production of a textile manufacturing unit is monitored by a plurality of sensors that are mounted on a plurality of production positions of the textile manufacturing unit. The plurality of sensors continuously measure one or more parameters of a production process of the textile manufacturing unit and continuously quality data is generated (e.g. by the sensors and/or by an online quality monitoring device comprising the sensors) based on the measurements of the sensors. Data comprising quality data can be transmitted to an offline testing system, in particular an offline testing system comprising offline testing instruments. The data can be transmitted to the offline testing system continuously or only on a specific condition. The data can be transmitted to the offline testing system if a parameter is outside a pre-defined set of values for that parameter.

The offline testing system can determine if to transmit the data to a specific offline testing instrument, e.g. to make the data available to a user of that specific offline testing instrument.

According to some variants, data comprising quality data is continuously sent from an online quality monitoring device to a server. The server and/or a user thereof can analyse the received data and determine if a suspicious event has occurred, e.g. a parameter value that is outside a certain range of parameter values has been measured. In case a suspicious event is identified, the server and/or a user can send data concerning the suspicious event to an offline testing instrument. Based on the data available at to the server, the server and/or a user can decide to which offline testing instrument which data should be sent. The server and/or the user can also initiate that a sample should be taken from the production position at which the suspicious event has occurred. For example, an indication means can indicate that a sample from a specific production position should be taken. The sample can be taken by a user or automatically by a robot. The sample can be taken to an offline testing instrument to which data concerning the suspicious event was sent. At the offline testing instrument an offline analysis of the sample can be conducted. Based on the result of the offline testing data and possibly based on the online data, possible sources of a problem and/or proposals on how to solve a problem can be identified. The identification can be made by a computer of the offline testing instrument, by the server and/or by a user using the offline testing instrument and/or accessing the server. Information concerning possible sources of the problem and/or proposals on how to solve the problem can be provided to a user and/or an automatic system. The information can be provided to a user e.g. by a displaying a text, icon or image indicating the possible sources of the problem and/or proposals on how to solve the problem.

According to some variants, the system can notify a user if a defect (i.e. that a parameter is outside a pre-defined set of values) occurs, thereby drawing his attention, e.g. immediately, to the production position in question and/or to samples of yarn produced therein. For example, the user can, e.g. immediately, analyse the cause of the defect that he was notified about by the online quality monitoring device, e.g. by analysing a sample from the respective production position in one or more offline testing systems. Based on such an analysis, the user e.g. can initiate suitable corrective actions.

According to some variants, the data is collected by a data collecting unit, the data of multiple data collection units is collected by a data receiving unit and the data is transmitted from the data receiving unit to the one or more offline testing instruments of the offline testing system.

According to some variants, a sample of yarn is gathered from a production position. For example, a sample of yarn can be collected periodically. For example, a sample of yarn can be collected automatically upon an indication that a parameter that is outside the pre-defined set of values has been measured. For example, a sample of yarn can be collected by a user upon an indication that a parameter that is outside the pre-defined set of values has been measured.

According to some variants, a sample of yarn can be collected based on a decision, e.g. by a user and/or a computer (e.g. a computer of a server). Such a decision can be based on quality data collected by a system for yarn production monitoring and transmitted to a user and/or a computer. In some variants, the occurrence of a fault is indicated to a user, e.g. by indications means (light bulb, smart phone etc.). In reaction to such indication, the user can take a decision (e.g. to take a sample and to analyze a sample in one or more offline testing instrument).

According to some variants, a sample of yarn of a production position in which a parameter that is outside the pre-defined set of values had been measured, is analyzed using at least one of the one or more offline testing instruments. The analysis can concern one or more features of yarn. The analysis can concern evenness of the yarn, thick places of the yarn, thin places of the yarn, neps in the yarn, objectable yarn faults, hairiness of the yarn, yarn count, twist of the yarn and/or yarn classification distribution.

According to some variants, based on the transmitted data and on the result of the analysis using at least one of the one or more offline testing instruments, a cause that has caused the parameter to be outside the pre-defined set of values is searched for and/or identified.

According to some variants, based on the transmitted data and on the result of the analysis using at least one of the one or more offline testing instruments, a measure to remedy the cause that has caused the parameter to be outside the pre-defined set of values is searched for and/or identified.

According to some variants, the data transmitted to an offline testing system is received by a server of the offline testing system. Results of an analysis using at least one of the one or more offline testing instruments can also be received by the server. In this case, the information is centralized on the server and a user and/or computer can take decisions (e.g. which part of textile manufacturing unit should be attended to) based on the centralized information. For example, a user can accessed the centralized information via terminal of the server and/or a mobile device wirelessly connected to the server.

According to some variants, the data is transmitted to one or more offline testing instruments of the offline testing system. For example, the data can be transmitted via a centralized server unit.

According to some variants, the data transmitted to the offline testing system comprises machine related data. The machine related data can for example comprise data that allows to associate certain parts of the data to a specific textile manufacturing unit and/or to a specific production position (e.g. a machine number and/or a position number).

According to some variants, an identified cause of a fault (i.e. an occurrence of a parameter being outside the pre-defined set of values) is indicated to a user. The fault can e.g. be indicated by indication means. The fault can e.g. be indicated in form of digital information and/or light signals.

According to some variants, a periodic fault is indicated to a user. A periodic fault is a periodic occurrence of a parameter being outside a pre-defined set of values. The periodic fault can be indicated by a light signal.

According to some variants, the indication of a fault, in particular of a periodic fault, comprises displaying a text, an icon and/or an image. The text, icon and/or image can be related to the component of a textile manufacturing unit, in particular a component which is causing the fault. For example, if a front delivery roller of 30mm diameter is defective, it may cause a periodic fault with periodicity π·30mm ≈ 94mm. If the system (e.g. an evenness tester) finds a suitable fault of this specific periodicity, the system may identify the front delivery roller as a possible cause for this periodic fault. The system can indicate this finding to a user by displaying e.g. an icon representing a front delivery roller and the number of the production position at which the fault has occurred. The user will then know that he has to check the front delivery roller of this specific production position.

According to some variants, an online quality monitoring device alerts a user in case that a component of a textile manufacturing unit causes a periodic fault, e.g. by generating a text, and icon or an image of the component of the component causing the periodic fault.

According to some variants, faults and/or the cause of faults, in particular periodic faults and/or the cause of periodic faults, are indicated by different light effects. For example, the different light effects can be different light colours and/or different lighting patterns, such as stable glowing, flashing or flashing at different frequencies.

According to some variants, data from the data receiving unit can be transferred to a centralized server.
Additionally, data from offline laboratory instruments can also be transferred to the centralized server. This can allow to perform a detailed analysis on the centralized server, e.g. to correlate the online data and offline data at the centralized server. This can allow the centralized server and/or a user thereof to identify possible causes of a problem and/or possible remedies for a problem. The results of such an analysis can also e.g. be used for further investigations of quality parameters and/or investigation of production fault causes. The results of such an analysis can also e.g. be saved and e.g. used for future investigations of quality parameters and/or investigation of production fault causes.

According to some variants, a centralized server collects data from a data receiving unit and transfers the collected data to offline testing instruments.

According to some variants, an online quality monitoring device can find a parameter outside the allowable limits (which can e.g. be different for different production positions) and provide information concerning the machine details, the production position, the yarn count, the linear density and/or quality parameters of the textile material. This information can be transferred from the online quality monitoring device e.g. to the relevant offline testing instruments. This can support a user in interpreting the details he finds by testing samples of the textile material of the faulty production position in the relevant offline testing instruments. The offline testing instruments can comprise computers and the data transferred from the online monitoring system to the offline instruments can be processed by such computers.

According to some variants, for example the following can occur: If an unacceptable thin fault in a textile material is identified by an online quality monitoring system and/or an offline testing system, details like the machine number, the spindle number, the linear density and/or quality parameters of the textile material are transmitted to the relevant online laboratory instrument(s).

Because thin faults can be measured in an evenness tester as well as in a yarn classification system, but (because thin faults also cause low strength) also in a tensile tester, the data from the online monitoring system can e.g. be transferred to all the three offline instruments. Once the data is transmitted, the user can evaluate in the offline system the root cause of the defect. In case of a thin place, user can e.g. analyze a spectrogram of textile material taken from the faulty position to identify whether periodically thin parts occur in the textile material. In case of such a periodic occurrence, the offline testing instrument (and/or an analysis therewith) can allow identifying the component of the textile manufacturing unit (e.g. a ring frame) responsible for the periodically occurring thin parts. Thus, such an integrated system can support a user and/or a machine to attend to the root cause of the defect in short time.

According to some variants, integration of online data and offline data includes intelligent analysis. For example, data concerning thick places of a textile material generated by the online quality monitoring system together with a spectrogram data generated by an offline evenness tester can be used to find the component of the machinery which causes a fault. Thus, by combining online data and offline data the source of a fault and/or possible remedies can be identified. Collecting data concerning the time at which a certain deviation occurred can also support to make future failure analysis of machinery components and/or faults.

A variant of the invention provides a system for integrating the online monitoring device of a textile manufacturing unit with the offline testing instruments wherein the online quality monitoring device comprises of plurality of sensors mounted on plurality of production positions that continuously generate quality data of the production process and transmits the quality data whenever they exceed a pre-set quality parameter to the offline laboratory testing instruments thereby benefiting the user to pay immediate attention to the samples or production positions that deviate the set quality standard and enable immediate analysis of the cause of such defects signalled by the online monitoring devices, for initiating suitable corrective action. According to a variant, the system transfers the online quality data from the textile manufacturing unit (e.g. like a ring spinning) to the offline laboratory testing instruments such as evenness tester, tensile tester, yarn classification system and/or twist tester. Thereby the system can provide useful data to those offline instruments, thus facilitating the user in making a detailed analysis of samples of textile materials of production position where parameters were found to be outside certain limits. The system can comprise a plurality of sensors mounted at each of production positions to continuously measure the quality of textile material delivered from the rollers, a data collection unit available at each machine where the measured data is transferred for data collection, a data receiving unit that collects data from multiple data collection units of each textile machine and means for transferring the data to the offline laboratory instruments for further analysis whenever the transmitted data fails the pre-set quality standard. The data from the data receiving unit can directly be sent to a central server and the data from the offline laboratory instruments can also be transmitted to the same server where a detailed analysis to correlate online and offline data can be carried out for further investigation of cause of the deviation from the pre-set quality standard. A centralized server can collect the data from a data receiving unit and transmits them to offline instruments for further analysis. The data transmission can be carried out through wired technologies such as RS 232, RS 422, TCP/IP or CAN (Controller Area Network) or through wireless technologies like Wi-Fi or Blue Tooth. The offline laboratory testing instruments can comprise an evenness tester, tensile tester, yarn classification system and twist tester for measuring quality of the textile material. The fault in textile material can be identified by an online monitoring system of a textile machine and details like machine number, spindle number, linear density and quality parameters of the textile material can be transmitted to the relevant offline instrument(s). The system can enable the user to evaluate in the offline system the root cause of the defect. The defective machine or portion of the defective machine can intimated in any form to a machine user such as a digital information or as light signals. E.g. a light indication device on the online monitoring device can alert the user whenever a component in the machine causes a periodic fault. Such a light indication device can e.g. generate a text, an icon or an image of a component of the machinery causing the defect or fault, thereby alerting the technicians in the department to attend the defective component immediately to set right the position. The defects detected by the online monitoring device can be signalled by different colour lamp indicators with stable glowing, flashing or flashing at different frequencies to denote the various conditions of the textile material. Defects detected and indicated visually by the online monitoring device can be transmitted to the offline laboratory testing instruments and represented visually through a light indication device or color lamp indicators. Another variant of the invention provides a method for integrating online monitoring device of a textile manufacturing unit with the offline testing instruments wherein the sensors of the online monitoring device mounted on plurality of production positions continuously generates data on the quality parameter of the production process and transfers the generated data whenever they deviate from the pre-set quality standard to the offline laboratory testing instruments to enable the user to pay immediate attention to the sample or production positions that deviated from the pre-set quality standard and to make analysis of the cause of such defects signalled by the online monitoring devices for taking the necessary corrective measures. The data generated by the online monitoring device can first be collected at a data collecting unit and the data from multiple data collecting units can then be collected in a data receiving unit from which the data can be transmitted to the offline testing instruments for analysis. The data collected at a data receiving unit and the data generated by an offline testing system can both be transmitted to a central server for analysis of a quality of a textile materials. Such a centralized server unit can collect the data from the data receiving unit and transmit it to instruments for offline laboratory testing for further analysis. The data can be transmitted through wired technologies such as RS 232, RS 422, TCP/IP or CAN (Controller Area Network) or through a wireless technology like Wi-Fi or Blue Tooth. The offline laboratory testing instruments can comprise an evenness tester, a tensile tester, a yarn classification system and a twist tester for measuring the quality of testing material. The fault in textile material can be identified by an online monitoring system of a textile machine and details like machine number, spindle number, linear density and quality parameters of the textile material can be transmitted to the relevant offline testing instrument(s). This can allow a user to evaluate the root cause of the defects from the readings of the offline testing instruments. The defective machine or portion of the defective machine causing fault can be identified and intimated in any form to the machine user such as by a digital information or by light signals. A light indication device on the online monitoring device can alert the user whenever a component in the machine is causing a periodic fault. The light indication device can generate a text, an icon or an image of the component of the machinery causing the periodic fault, thereby alerting the technicians in the department to attend the defective component immediately to set right the position. The defects detected by the online monitoring device can be signalled by different colour lamp indicators with stable glowing, flashing or flashing at different frequencies to denote the various conditions of the textile material. The defect information detected and indicated visually by the online monitoring device can be transmitted to the offline laboratory testing instruments and can be represented visually through a light indication device or color lamp indicators.

The invention further concerns an apparatus comprising means adapted to execute any of the methods disclosed herein.

The subject matter of the invention will be explained in more detail in the following text with reference to exemplary embodiments which are illustrated schematically in the attached drawings.
- Figure 1: shows an assembly comprising a system for yarn production monitoring, wherein the sensors of the online quality monitoring device are mounted on production positions of a textile manufacturing unit;
- Figure 2: shows a system for yarn production monitoring comprising a server.
- Figure 3: shows a sensor comprising an indication device.

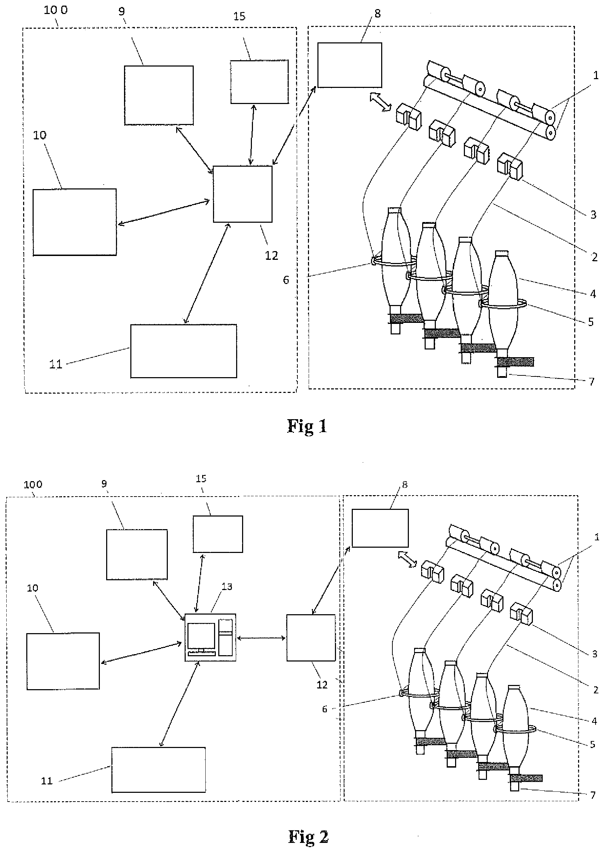

Figure 1 shows a system yarn production monitoring, wherein sensors 3 are mounted at production positions of a textile manufacturing unit.

The system for yarn production monitoring comprises an online quality monitoring device and an offline testing system (100). The online quality monitoring device comprises one or more sensors 3, which are adapted to be mounted at a production position of a textile manufacturing unit and to continuously measure a parameter of a production process of a production position at which they are mounted. The online quality monitoring device is adapted to generate quality data of a production process using results of measurements by the sensors 3. The system can be adapted to transmit data comprising quality data from the online quality monitoring device to the offline testing system (100). The system can be adapted to e.g. transmit such data continuously (e.g. to a server of the offline testing system). The system can be adapted to e.g. transmit such data if a parameter is outside a pre-defined set of values. Such a system is an integrated system, as it integrates online and offline measurement units.

Figure 1 shows four sensors 3 of an online quality monitoring device. Each of the four sensors 3 is mounted at a production position of a textile manufacturing unit at which a textile material (e.g. yarn) is produced.

As shown in Figure 1, the textile material 2 can be produced delivered from a pair of drafting rollers 1 and wound on a cop 4 on a spindle 7 by a traveller 6 that is rotating over a ring 5. In the example of Figure 1, the sensors 3 of the online quality monitoring device are arranged along the spinning path of each production position, between the drafting rollers 1 and the traveller 6. The production positions can additionally comprise lappet hooks (not shown) for guiding the yarn, and the sensors 3 can be mounted at such a lappet hook, e.g. directly above and/or beneath the lappet hook. The sensors 3 can be adapted to measure one or more parameters of the production process. The measured parameters can relate to quality data, such as e.g. U%, CV%, CV% at various cut lengths, thin places, thick places, neps, objectionable yarn faults, yarn hairiness, yarn count and/or yarn twist. The results of the measurements are and/or allow to generate quality data of the production process. The quality data can be used to find defects in the production process.

The embodiment of Figure 1 additionally comprises a data collection unit 8. The data collection unit 8 can be comprised in the online quality monitoring device or independent thereof. In the example of Figure 1, the data collection unit 8 is comprised in the online quality monitoring device. A data collection unit 8 can be adapted to collect data from all the sensors 3 of the online quality monitoring device that are mounted on a specific textile manufacturing unit. In a setting comprising multiple textile manufacturing units, the system can comprise multiple data collection units 8, and to each textile manufacturing unit a data collection unit 8 can be associated. In particular, there can be exactly one data collection unit 8 per textile manufacturing unit.

The embodiment of Figure 1 additionally comprises a data receiving unit 12. The data receiving unit 12 can be comprised in the offline testing system 100 or independent thereof. In the example of Figure 1, the data receiving unit 12 is comprised in the offline testing system 100. A data receiving unit 12 can be adapted to collect data from various data collection units 8. The data receiving unit 12 can be adapted to transmit data to one or more offline testing instruments of the offline testing system 100, e.g. to the computers of such offline testing instruments. The system can comprise two or more data receiving units 12.

The offline testing system 100 can comprise one or more offline testing instruments. In the example of figure 1, the offline testing system 100 comprises an evenness tester 9, a tensile tester 10, a yarn classification system 11 and a twist tester 15.

The system can be adapted so that, if a parameter is outside a pre-defined set of values, the online quality monitoring device transmits quality data and possibly other data (e.g. the number of the production position at which the problem has occurred) to the offline testing system 100. In the example if Figure 1, the data of a textile manufacturing unit is collected by a data collection unit 8 and the data collected by the collection units 8 is collected by the data receiving unit 12, which transmits the data to offline testing instruments of the offline testing system 100. For example, multiple data collection units 8 can be associated with multiple textile manufacturing units and the data of the multiple data collection units 8 can be collected by a single data receiving unit 12.

Figure 2 shows a system and a part of a textile manufacturing unit similar to that of Figure 1. In the example of Figure 2, the system further comprises a server 13, e.g. a centralized server. The server 13 can be adapted to receive data from one or more data receiving unit 12 and transfer the data to offline laboratory instruments (e.g. to computers thereof) at which analysis of samples can be performed. The server 13 can be adapted to coordinate testing of samples at the offline laboratory instruments (e.g. by deciding if to send data to a specific offline laboratory instrument). The sever 13 can be adapted to collect data of testing of samples of one or more offline testing instruments. The sever 13 can be adapted to analyse data collected from the online quality monitoring device (e.g. to determine if an offline testing should be conducted). The sever 13 can be adapted to analyse data collected from the online quality monitoring device and data from some of the offline laboratory instruments (e.g. to determine the cause of and/or possible remedies for a fault).

The system can be adapted to indicate an occurrence of a fault and/or a cause of a fault and/or a possible remedy for a fault to a user and/or a machine. For such indication, the system, in particular the online quality monitoring device, can comprise indication means in form of one or more indication devices 14. In some embodiments of a combination of the system with one or more textile manufacturing units, to each production position an indication device 14 is associated (e.g. mounted on that production position, for example as part of the senor 3). This can e.g. be used to indicate an occurrence in a specific production position. This can allow a user to quickly identify in which production position a fault has occurred and e.g. quickly collect a sample of yarn from this production position, which can then be analysed in greater details, e.g. in offline laboratory instruments of the offline testing system 100.

In some embodiments, the system can be adapted to transfer data concerning the cause of a fault and/or possible remedies to one or more indication devices 14. An indication device 14 can e.g. be suited to indicate different causes/remedies by different signals, e.g. by different colors or different flashing patterns of a light indication device. Indicating the cause and/or a possibly remedy at a specific production position can support the staff in quickly correcting the fault and to resume un-faulty production at that production position.

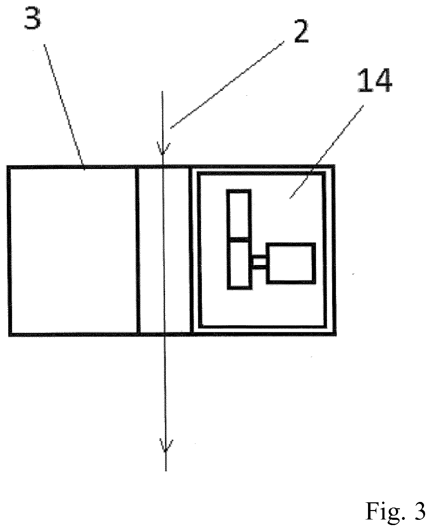

Figure 3 shows an example of an indication device 14. In the example of Figure 3, the indication device 14 is comprised in a sensor 3 of the online quality monitoring device. As also shown in Figure 3, the sensor 3 and the indication device 14 can be arranged at proximity to the textile material path of a production position.

## Claims

1. A system for yarn production monitoring, comprising
- an online quality monitoring device, and
- an offline testing system (100)
- wherein the online quality monitoring device comprises a sensor (3),
∘ wherein the sensor (3) is adapted to be mounted at a production position of a textile manufacturing unit,
∘ wherein the sensor (3) is adapted to continuously measure a parameter of a production process of a production position at which it is mounted,
∘ wherein the online quality monitoring device is adapted to generate quality data of a production process using results of measurements by the sensor (3); and
- wherein the system is adapted to transmit data comprising quality data from the online quality monitoring device to the offline testing system (100).

2. The system of claim 1, wherein the system is adapted to transmit data comprising quality data from the online quality monitoring device to the offline testing system (100) if a parameter is outside a pre-defined set of values.

3. The system of one of the preceding claims, comprising a data collection unit (8) that is adapted to collect data from a sensor (3),
- in particular from two or more sensors (3),
- in particular wherein the data collection unit (8) is adapted to collect data from all the system's sensors (3) mounted at a single textile manufacturing unit, and/or
- in particular wherein the system is adapted to transmit the data from the online quality monitoring device to the offline testing system (100) via the data collection unit (8).

4. The system of one of the preceding claims, comprising a data receiving unit (12) that is adapted to receive data from one or more data collection units (8), and comprises means for transmitting data to the offline testing system (100),
- in particular wherein the system is adapted to transmit the data from the online quality monitoring device to the offline testing system (100) via the data receiving unit (12).

5. The system of one of the preceding claims, wherein the offline testing system (100) comprises an offline testing instrument (9,10,11,15),
- in particular an offline testing instrument that comprises an evenness tester (9), a tensile tester (10), a yarn classification instrument (11) and/or a twist tester (15).

6. The system of one of the preceding claims, wherein the offline testing system (100) comprises a server (13),
- in particular wherein the system is adapted to transmit data comprising quality data from the online quality monitoring device to the server (13) of the offline testing system (100),
- in particular wherein the server (13) is adapted to analyse data provided by the online quality monitoring device and data provided by the offline testing system (100), and/or
- in particular wherein the server (13) is adapted to collect data from the data receiving unit (12) and adapted to transmit the collected data to an offline testing instrument (9,10,11,15).

7. The system according to one of the preceding claims, wherein the data that the system is adapted to transmit comprises a machine number, a spindle number, and/or a linear density.

8. The system according to one of the preceding claims, wherein the system comprises indication means (14),
- in particular wherein the system is adapted to activate the indication means (14) if a parameter is outside a pre-defined set of values,
- in particular wherein the indication means (14) comprise digital information means and/or light indication means.

9. A combination comprising
- a textile manufacturing unit comprising a plurality of production positions, and
- a system according to one of claims 1 to 8,
∘ wherein the online quality monitoring device comprises a plurality of sensors (3),
- wherein the plurality of sensors (3) of the online quality monitoring device is mounted on the plurality of production positions,
- wherein the plurality of sensors (3) is adapted to continuously generate quality data of the production process.

10. The combination of claim 9,
- wherein the plurality of productions positions comprises rollers (1) for delivering the yarn being produced, and
- sensors (3) of the plurality of sensors are mounted at each of the production positions,
∘ wherein the sensors (3) are adapted to continuously measure the quality of the yarn (2) delivered from the rollers (1).

11. A method for yarn production monitoring,
- wherein a production of a textile manufacturing unit is monitored by a plurality of sensors (3) that are mounted on a plurality of production positions of the textile manufacturing unit,
- wherein the plurality of sensors (3) continuously measure one or more parameters of a production process of the textile manufacturing unit,
- wherein continuously quality data is generated based on the measurements of the sensors (3), and
- wherein data comprising quality data is transmitted to an offline testing system (100).

12. The method according to claim 11, wherein data comprising quality data is transmitted to an offline testing system (100) comprising one or more offline testing instruments (9,10,11,15) if a parameter is outside a pre-defined set of values.

13. The method according to claims 11 or 12,
- wherein the data is collected by a data collecting unit (8),
- wherein the data of multiple data collection units (8) is collected by a data receiving unit (12), and
- wherein the data is transmitted from the data receiving unit (12) to the one or more offline testing instruments (9,10,11,15) of the offline testing system (100).

14. The method according to one of claims 11 to 13,
- wherein a sample of yarn of an production position, in which a parameter that is outside of a pre-defined set of values has been measured, is analyzed using at least one of the one or more offline testing instruments (9,10,11,15),
∘ in particular wherein the analysis concerns at least one of the following features of yarn:
▪ evenness of the yarn,
▪ thick places of the yarn,
▪ thin places of the yarn,
▪ neps in the yarn,
▪ objectable yarn faults,
▪ hairiness of the yarn,
▪ yarn count,
▪ twist of the yarn, and/or
▪ yarn classification distribution.

15. The method according to claim 14,
- wherein, based on the transmitted quality data and on the result of the analysis using at least one of the one or more offline testing instruments (9,10,11,15),
∘ a cause that has caused the parameter to be outside the pre-defined set of values is identified; and/or
∘ a measure to remedy the cause that has caused the parameter to be outside the pre-defined set of values is identified.

16. The method according to one of claims 11 to 15,
- wherein the data transmitted to an offline testing system (100) is received by a server (13) of the offline testing system; and
- wherein results of an analysis using at least one of the one or more offline testing instruments (9,10,11,15) are received by the server (13).

17. The method according to one of claims 11 to 16,
- wherein the data is transmitted to one or more offline testing instruments (9,10,11,15) of the offline testing system (100),
∘ in particular wherein the data is transmitted via a server (13).

18. The method according to one of claim 11 to 17,
- wherein the cause of faults, in particular of periodic faults, are indicated by different light effects,
∘ in particular by different light colours; and/or
∘ in particular by different light patterns, such as stable glowing, flashing or flashing at different frequencies.
